# EUROPEAN PATENT SPECIFICATION

(11) **EP 2 085 024 B1**
(45) Date of publication and mention of the grant of the patent: **09.11.2011**
(21) Application number: 09001093.5
(22) Date of filing: 27.01.2009
(51) Int. Cl.: A61B 5/00

(54) **Adaptor device and medical apparatus system**
Adaptervorrichtung und medizinisches Vorrichtungssystem
Appareil d'adaptateur et système de dispositif médical

(30) Priority: 29.01.2008 JP 2008017774
(43) Date of publication of application: 05.08.2009
(73) Proprietor: Olympus Medical Systems Corp., Tokyo 151-0072 (JP)
(72) Inventor: Kagawa, Ryohei, Tokyo 151-0072 (JP)
(74) Representative: von Hellfeld, Axel

(56) References cited:
- EP-A- 0 735 498
- US-A1- 2005 047 055
- US-A1- 2006 122 529
- US-B1- 6 712 762

## Description

### Background of the Invention

### Field of the Invention

The present invention relates to a technique for insulating a medical apparatus from a computer when data is transferred.

### Description of the Related Art

A capsule endoscope system is commonly constituted by a portable medical apparatus (i.e., a reception device), an inspection-data-debug-use computer (i.e., a work station), and a data-communication-use medium (such as a communication cable) for interconnecting the aforementioned components.

Therefore, if a patient is equipped with a portable medical apparatus that is in contact with her/his skin, such as the antenna pad of the reception device, the portable medical apparatus must be electrically insulated securely from a commercial power source and an electrical ground (GND). One method for securing electrical insulation is a method of applying an electrical insulation measure through the communication line between the reception device and work station.

Meanwhile, the techniques related to the present invention proposal include Laid-Open Japanese Patent Application Publication Nos. 2006-75244 and 2005-304513.

US 2006/0122529 A1 discloses a system for measuring a patient's AC and DC neural response signals using electrodes connected to an amplifier unit worn by the patient. The data from the electrodes is converted to digital form, buffered and amplified, and transmitted to a microprocessor for storage in a memory. The microprocessor transmits data to and receives data from a controller via fiber optic transmission and receiving circuits. The controller receives fiber optic data from the microprocessor of the wearable amplifier, buffers the data, and transmits the data to a computer (PC) via a USB connection. The amplifier is electrically isolated from all other electrical devices. Because safety issues arise from using an AC power outlet, and because isolation transformers are a source of electrical and magnetic noise, a rechargeable battery is used to power the amplifier unit. During recharging time, the patient amplifier unit is internally disconnected using relays for safety and noise considerations. Because current consumption is a concern, the battery needs to last at least a working shift.

EP 0735498 A1 discloses a portable device for continuously acquiring medical data from a plurality of sensors adapted for attachment to a patient via a docking station in communication with a central workstation via a LAN connection. The docking station electrically isolates paths connected to the monitors. A cable provides power and data communications with the portable device, and the cable may be directly interfaced to the LAN network. The docking station supplies power to the portable monitor to recharge a battery inside the portable monitor when the monitor is docked on the docking station.

US 2005/0047055 A1 discloses a combined power source and signal coupler for a patient-connected monitor, including a docking station and a portable device capable of docking with the docking station, each of which include a power coupler and an electrically isolated data transducer. The monitor and docking station are connected to a single power supply. Further, the monitor communicates with the docking station via an RF connection when undocked. When the monitor is docked, optical transducers in the monitor provide two-way communication with optical transducers in the docking station. Further, the docking station communicates with a central controller via a LAN connection coupled with the optical transducers of the docking station.

### Summary of the Invention

The invention is directed to the problem of insulating a medical apparatus from a computer when data is transferred.

This problem is solved by an adaptor device according to claim 1 and a medical apparatus according to claim 10.

An adaptor device according to the present invention is connected to both a portable medical apparatus and a computer that exchanges data with the portable medical apparatus, comprising:
a first interface connected to the computer and which communicates at a first communication speed with the computer;
a second interface connected to the portable medical apparatus and which communicates at a second communication speed with the portable medical apparatus;
a communication control unit for adjusting the speed between a first signal exchanged at the first communication speed by way of the first interface and a second signal exchanged at the second communication speed by way of the second interface;
a first insulation unit equipped between the second interface and the communication control unit and which electrically insulates between a terminal on the second interface side and a terminal on the communication control unit side, for outputting, to the communication control unit, the second signal input from the second interface; and
a second insulation unit equipped between the second interface and the communication control unit and which electrically insulates between a terminal on the second interface side and a terminal on the communication control unit side, for outputting, to the second interface, a signal input from the communication control unit.

Further, a medical apparatus system according to the present invention is constituted by a portable medical apparatus, a computer, and an adaptor device connecting the portable medical apparatus to the computer, wherein
the adaptor device comprises:
a first interface which is connected to the computer and which communicates at a first communication speed with the computer;
a second interface which is connected to the portable medical apparatus and which communicates at a second communication speed with the portable medical apparatus;
a communication control unit for adjusting the speed between a first signal exchanged at the first communication speed by way of the first interface and a second signal exchanged at the second communication speed by way of the second interface;
a first insulation unit equipped between the second interface and the communication control unit and which electrically insulates between a terminal on the second interface side and a terminal on the communication control unit side, for outputting, to the communication control unit, the second signal input from the second interface; and
a second insulation unit equipped between the second interface and the communication control unit and which electrically insulates between a terminal on the second interface side and a terminal on the communication control unit side, for outputting, to the second interface, a signal input from the communication control unit.

### Brief Description of the Drawings

Fig. 1 shows an outline of the connection between a human-body contacting portable medical apparatus and a work station when data is transferred, according to a first preferred embodiment;
Fig. 2 is a block diagram of an insulation adaptor 6 according to a second preferred embodiment;
Fig. 3 shows an outline of the connection between a human-body contacting portable medical apparatus and a work station when data is transferred, according to the second embodiment;
Fig. 4 is a block diagram of an insulation adaptor 6 according to the second embodiment (exemplary embodiment 1);
Fig. 5 is a block diagram of an insulation adaptor 6 according to the second embodiment (exemplary embodiment 2);
Fig. 6 shows an outline of the connection between a human-body contacting portable medical apparatus and a work station when data is transferred according to a third preferred embodiment;
Fig. 7 is a block diagram of a cradle 30 according to the third embodiment; and
Fig. 8 is a block diagram of a reception device 4a according to the third embodiment.

### Description of the Preferred Embodiments

An adaptor device according to the present invention can be connected to both a portable medical apparatus and a computer which exchanges data with the portable medical apparatus. The aforementioned adaptor device comprises a first interface, a second interface, a communication control unit, a first insulation unit, and a second insulation unit.

The first interface is an interface which is connected to the computer and which communicates at a first communication speed with the computer. The first interface is equivalent to a USB cable connector 18 according to, for example, a preferred embodiment of the present invention.

The second interface is an interface which is connected to the portable medical apparatus and which communicates at a second communication speed with the portable medical apparatus. The second interface is equivalent to an exclusive-use cable connector 11 or cradle connector 31 according to, for example, the embodiment of the present invention.

The communication control unit adjusts the speed between a first signal exchanged at the first communication speed by way of the first interface and a second signal exchanged at the second communication speed by way of the second interface. The communication control unit is equivalent to a communication control unit 15 according to, for example, the embodiment of the present invention.

The first insulation unit is equipped between the second interface and the communication control unit and is electrically insulated (also simply noted as "insulated" hereinafter) between a terminal on the second interface side and a terminal on the communication control unit side. The first insulation unit outputs, to the communication control unit, the second signal input from the second interface. The first insulation unit is equivalent to an insulation element 13 according to, for example, the embodiment of the present invention.

The second insulation unit is equipped between the second interface and the communication control unit and is electrically insulated between a terminal on the second interface side and a terminal on the communication control unit side. The second insulation unit outputs, to the second interface, a signal input from the communication control unit. The second insulation unit is equivalent to an insulation element 14 according to, for example, the embodiment of the present invention.

Here, a photo coupler can be used for the first insulation unit and second insulation unit. The second communication speed is in a range that is compatible with the photo coupler. The first communication speed is based on a communication protocol used for a general purpose computer equipped with at least a universal serial bus (USB).

Configuring as described above makes it possible to adjust a communication speed between a portable medical apparatus and a general purpose personal computer (PC) and to secure electrical insulation between the portable medical apparatus and PC.

Further, the communication control unit converts the communication speed of the second signal input by way of the first insulation unit into the first communication speed, which is higher than the second communication speed, and outputs the converted communication speed to the first interface. Further, the communication control unit converts the communication speed of the first signal input by way of the first interface into the second communication speed and outputs the converted communication speed to the second insulation unit.

Configuring as described above makes it possible to fill a gap in the communication speeds between the portable medical apparatus and the PC.

The adaptor device further comprises a first power supply unit and a second power supply unit.

The first power supply unit drives the first insulation unit and the communication control unit. The first power supply unit is a power supply circuit 17 or a power- supply-line-use terminal 18a for supplying the bus power of USB according to, for example, the embodiment of the present invention.

The second power supply unit is insulated from the first power supply unit and drives the second insulation unit. The second insulation unit is equivalent to a power supply circuit 12, an AC adaptor 20a, or a power supply circuit 22b according to, for example, the embodiment of the present invention.

Configuring as described above makes it possible to form an insulated line between the primary side and secondary side.

For example, if the first interface is an interface based on USB, the first power supply unit can use the power based on the bus power of the USB (e.g., the power supply circuit 17 or the power-supply-line-use terminal 18a). In this case, the second power supply unit uses a power supply (e.g., the power supply circuit 12) on the basis of the power supplied from the portable medical apparatus.

Further, if, for example, the first interface is an interface based on the USB, the first power supply unit can use a power supply based on the bus power of the USB (e.g., the power supply circuit 17 or the power-supply- line-use terminal 18a) In this case, the second power supply unit can adopt an insulated AC adaptor (e.g., the AC adaptor 20a) enabled to supply a commercial power source by being connected to the adaptor device.

Further, if, for example, the first interface is an interface based on the USB, the first power supply unit can use the power based on the bus power of the USB (e.g., the power supply circuit 17 or the power-supply- line-use terminal 18a). In this case, if the AC adaptor is an uninsulated AC adaptor enabled to supply commercial power by being connected to the adaptor device, the second power supply unit can use an insulated power supply circuit (e.g., the power supply circuit 22b) that adjusts the power supplied from the AC adaptor and supplies the second insulation unit with the adjusted power.

Note that the adaptor device can be configured as a cradle enabling the attachment of the portable medical apparatus. In this case, the adaptor device further comprises a charger unit.

The charger unit charges a rechargeable battery existing inside of the portable medical apparatus by way of the second interface. The charger unit is equivalent to a charger 32 according to, for example, the embodiment of the present invention. When the portable medical apparatus is attached, the charger unit supplies the rechargeable battery with power. In this event, the second power supply unit supplies the power by way of the second interface for driving the portable medical apparatus.

Configuring as described above enables the reception device to be charged while transferring data. Further, the reception device uses the AC power source as the drive power while being attached to the cradle, thereby making it possible to suppress the consumption of the built-in rechargeable battery in the reception device.

The following is a description, in detail, of a preferred embodiment of the present invention.

### <First Embodiment>

For the insulation of a communication line, there is no insulation element which is compatible with a communication protocol for a high-speed communication such as USB 2.0 and is equipped in a general purpose personal computer (PC). Due to this, a conventional technique needs to be used, in which case the following problem occurs.

If a wireless method such as a wireless LAN or a wireless USB is used for communication between a portable medical apparatus and a PC, a problem that is specific to the wireless communication occurs, such as a problem with the directivity of a communicating direction, the stability of communication, and a communication distance.

Further, a data communication is performed using a recording-medium-use cradle between a cradle and a PC with the recording medium inside of a portable medical apparatus removed. This requires the cumbersome task of detaching the recording medium, thus ushering in a decrease in usability. Further, incorporating a quick-release mechanism for a recording medium is disadvantageous for miniaturization of a portable medical apparatus (instead, a built-in recording medium is advantageous to miniaturization).

Meanwhile, if a portable medical apparatus or data-communication-use medium is equipped with a mechanical structure for preventing the connection of the data-communication-use medium to a medical apparatus while the apparatus is mounted on a patient, the equipping of, for example, a protrusion makes it difficult to guarantee the mechanical strength and decreases the productivity.

It is accordingly conceivable to equip a communication line between the portable medical apparatus and a PC with an insulation adaptor. In such a case, it is conceivable to use an insulation element within the insulation adaptor.

However, the communication protocol, such as USB 2.0, equipped in a general purpose PC is for a high-speed communication and therefore an insulation element compliant with such a high-speed communication does not exist.

In the meantime, when data is transferred from a portable medical apparatus to an insulation adaptor, a certain period of time is necessary for communication that is required to access the recording medium inside of the portable medical apparatus, and therefore the communication speed between the portable medical apparatus and insulation adaptor may not necessarily be high.

Therefore, the present embodiment provides an adaptor device capable of adjusting a communication speed between a portable medical apparatus and a PC and also of securing electrical insulation (also simply noted as "insulation" hereinafter) between the portable medical apparatus and PC.

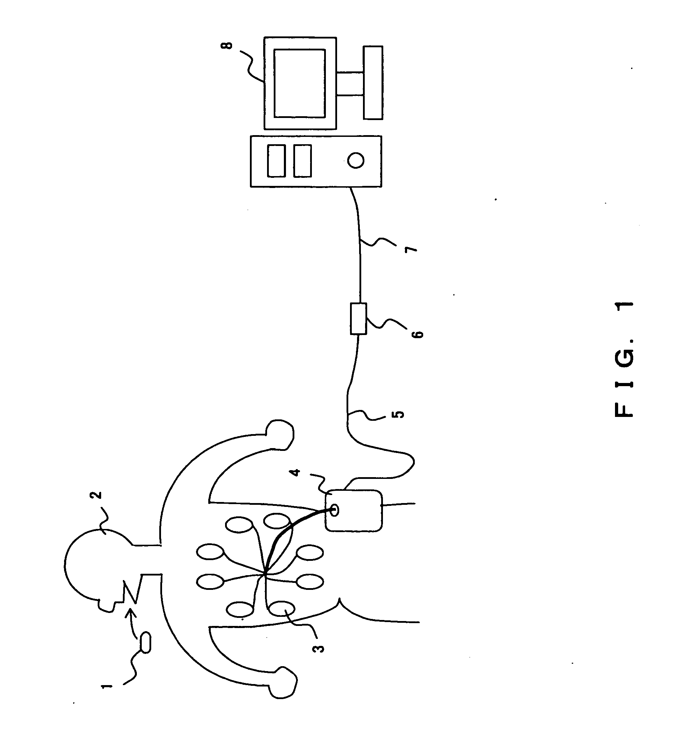

Fig. 1 shows an outline of the connection between a human-body contacting portable medical apparatus and a work station when data is transferred, according to the present embodiment. The present embodiment is described for the case of utilizing a reception device for receiving data picked up by a capsule endoscope as a human-body contacting portable medical apparatus.

At an endoscopic examination, pieces of data transmitted wirelessly from a capsule endoscope 1 that has been swallowed by the patient 2 are received at an antenna pad 3 and are stored piece by piece in the recording medium inside of the reception device 4, which is the human-body contacting portable medical apparatus. After ending the endoscopic examination, the data stored in the reception device 4 is transferred to a work station (WS) 8 by way of exclusive-use cable 5, insulation adaptor 6, and high-speed communication cable 7.

The high-speed communication cable 7 is compliant with a communication protocol, for example, USB and the like, adopted for a work station. The present embodiment uses a USB cable as an exemplary case of the high-speed communication cable 7, but the usage of USB is arbitrary.

The exclusive-use cable 5 is compliant with a communication protocol adopted for the reception device 4, and this communication protocol is commonly a communication standard for a slower speed communication than USB 2.0.

The insulation adaptor 6 is provided for insulating between the specific-use cable 5 and the USB cable 7 and also for stabilizing the communication between them.

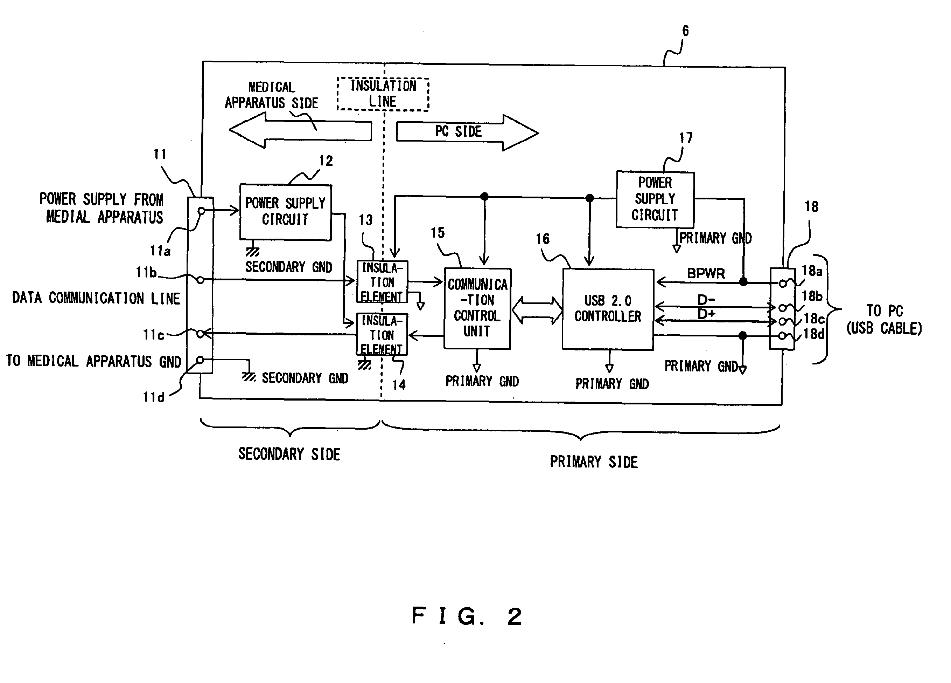

Fig. 2 is a block diagram of the insulation adaptor 6 according to the present embodiment. The insulation adaptor 6 comprises an exclusive-use cable connector 11, a power supply circuit 12, insulation elements 13 and 14, a communication control unit 15, a USB controller 16, a power supply circuit 17, and a USB cable connector 18.

The exclusive-use cable connector 11 is a connector used for connecting the exclusive-use cable 5 to the insulation adaptor 6. The exclusive-use cable connector 11 is equipped with a power supply-use terminal 11a, a data-communication-line-use input terminal 11b, a data-communication-line-use output terminal 11c, and a ground-line-use terminal 11d.

The power-supply-use terminal 11a is a terminal to which the power supplied from the reception device 4 is input. The data-communication-line-use input terminal 11b is a terminal used as the communication line for transferring data transmitted from the reception device 4. The data-communication-line-use output terminal 11c is a terminal used as the communication line for transferring data to be transmitted to the reception device 4. The ground-line-use terminal 11d is a terminal used for connections to the ground line of the reception device 4.

The USB cable connector 18 is a connector used for connecting the USB cable 7 to insulation adaptor 6. USB cable connector 18 is equipped with a power-supply-line-use terminal 18a, a D- line-use terminal 18b, a D+ line-use terminal 18c, and a ground-line-use terminal 18d.

The power supply circuit 12 adjusts the power supplied from the reception device 4 by way of the power-supply-use terminal 11a and supplies the insulation element 14 with the adjusted power. The power supply circuit 17 adjusts the USB bus power supplied by way of the power-supply-line-use terminal 18a and supplies the insulation element 13, communication control unit 15, and USB controller 16, all with the adjusted bus power.

The communication control unit 15 is a device used for converting a data communication speed. The USB controller is used for controlling the USB.

The insulation elements 13 and 14 are each an insulation element such as a photo coupler or a transformer. The present embodiment is configured to use a photo coupler as the insulation element; the usage thereof, however, is arbitrary. The photo coupler is constituted by a light-emitting element and a light-receiving element. The photo coupler, receiving an electric signal, is capable of converting the electric signal into light using the light-emitting element, receiving the light using the light-receiving element, and converting the light into an electric signal.

The power supply circuit 12, insulation element 14, and ground-line-use terminal 11d are connected to the ground GND on the secondary side (i.e., on the WS 8 side). The insulation element 13, communication control unit 15, USB controller 16, power supply circuit 17, and ground-line-use terminal 18d are connected to the ground GND on the primary side (i.e., the reception device 4 side). As a result, the insulation adaptor 6 is insulated in the part indicated by the dotted line shown in Fig. 2.

Next is a description of an outline of the operation of the insulation adaptor 6. To begin with, a description is provided for the case of transferring data from the reception device 4 to WS 8. A data signal (i.e., an electric signal) is input from the reception device 4 to insulation element 13 by way of the data-communication-line-use input terminal 11b. The data signal input to the insulation element 13 is output to the communication control unit 15. The communication control unit 15 adjusts the communication speed of the input data signal to convert it into a USB-use communication speed. The data signal output from the communication control unit 15 is input into the USB controller 16. The USB controller 16 transmits the data signal to the WS 8 by way of the D- line-use terminal 18b or D+ line-use terminal 18c.

Next is a description of the case of transferring data to the reception device 4 from WS8. A data signal (i.e., an electric signal) is input into the USB controller 16 by way of the D- line-use terminal 18b or D+ line-use terminal 18c. The USB controller 16 transmits the data signal to the communication control unit 15. The communication control unit 15 adjusts the communication speed of the input data signal to convert it into an exclusive-use cable-use speed. The data signal output from the communication control unit 15 is input into the insulation element 14. The data input into the insulation element 14 is output to the reception device 4 by way of the data-communication-line-use output terminal 11c.

The present embodiment attains isolation by means of a line, thereby enabling a more stable data communication than a case of isolation attained by means of a wireless. Further, the adopting of a method incorporating a recording medium inside of a medical apparatus makes it possible to secure the isolation while ensuring the communication between the medical apparatus and the WS. Therefore, the cumbersome task of detaching the recording medium from the medical apparatus just for reading the content of the recording medium is no longer required. Further, it is no longer necessary to equip a medical apparatus or an insulation adaptor with an insulation mechanism so as to prevent a connection, which improves the miniaturization and secures physical strength. Further, it is not necessary to equip a portable medical apparatus with an insulation mechanism, further improving the miniaturization. Further, it is also not necessary to equip the PC with an insulation mechanism and therefore the insulation can be attained only by an insulation adaptor. In the meantime, if the usage is limited to a state in which the reception device 4, WS 8, and insulation adaptor 6 are interconnected, a sufficient capacity of the data-recording-use medium may be small.

### <Second Embodiment>

The present embodiment is described for the case of attaching an AC adaptor to an insulation adaptor. Note that the same component sign is assigned to the constituent component described for the first embodiment and the description is not provided here.

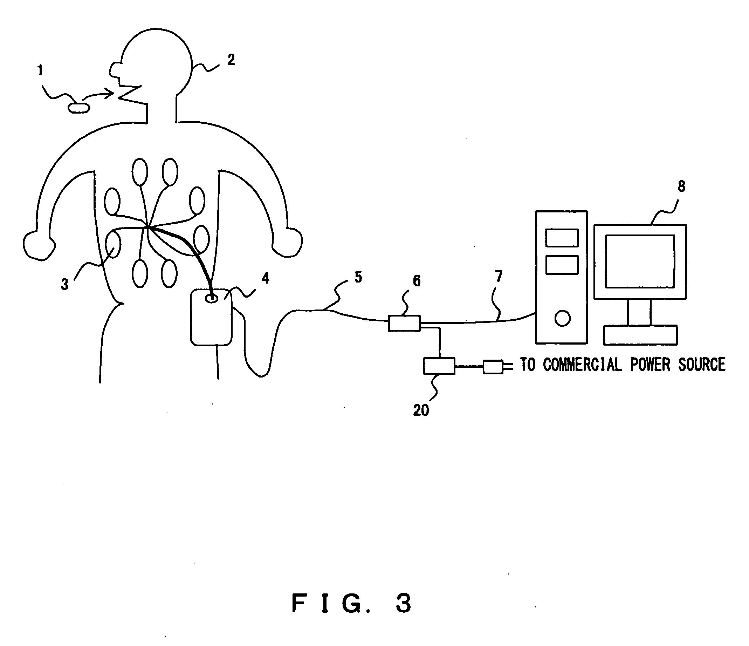

Fig. 3 shows an outline of the connection between a human-body contacting portable medical apparatus and a work station when data is transferred, according to the present embodiment. As with the other embodiment, the present embodiment is also described for the case of utilizing a reception device used for receiving data picked up by a capsule endoscope as a human-body contacting portable medical apparatus.

In the configuration shown in Fig. 3, an AC adaptor 20 is attached to an insulation adaptor 6. The AC adaptor 20 connected to the commercial power source supplies the insulation adaptor 6 with power on the basis of the commercial power source so as to drive the circuit inside of the insulation adaptor 6. Furthermore, the AC adaptor 20 supplies the reception device 4 with power via the insulation adaptor 6.

As a method of attaching the AC adaptor 20 to the insulation adaptor 6, it is conceivable to use a method of equipping the AC adaptor 20 with an insulated power supply circuit and, instead of the aforementioned method, to use a method of equipping the inside of the insulation adaptor with an insulated power supply circuit. Accordingly, both of these methods will be described in the following exemplary embodiments.

### [Embodiment 1]

Exemplary embodiment 1 is described for the method of equipping the AC adaptor with an insulated power supply circuit.

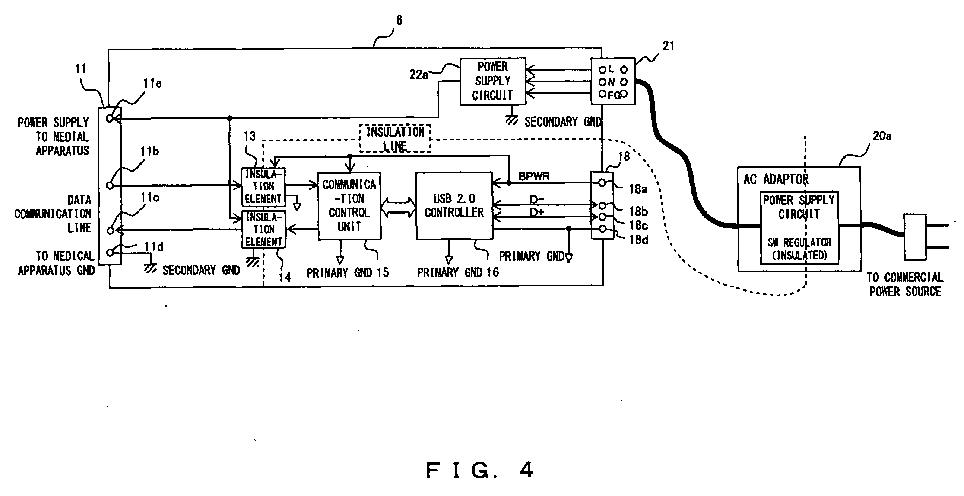

Fig. 4 is a block diagram of an insulation adaptor 6 according to the present embodiment (exemplary embodiment 1). The insulation adaptor 6 comprises an exclusive-use cable connector 11, insulation elements 13 and 14, a communication control unit 15, a USB controller 16, a power supply circuit 22a, a USB cable connector 18, and an AC adaptor input connector 21.

The exclusive-use cable connector 11 is equipped with a data-communication-line-use input terminal 11b, a data-communication-line-use output terminal 11c, a ground-line-use terminal 11d, and a power-supply-use terminal 11e. The power-supply-use terminal 11e is a terminal used so that the power supply circuit 22a can supply a reception device 4 with power.

The present embodiment is configured such that the AC adaptor 20a uses an insulated power supply circuit (e.g., an insulated switching (SW) regulator or the like). In this case, the AC adaptor input connector 21 of the insulation adaptor 6 is equipped with a specially formed connector so as to prevent an erroneous insertion of another uninsulated power supply adaptor.

The power supply circuit 22a adjusts the power supplied from the AC adaptor 20a via the AC adaptor input connector 21, supplies the insulation element 14 with the adjusted power, and further supplies the reception device 4 with the adjusted power via the power-supply-use terminal 11e.

The present embodiment is configured to supply the power of the primary side (noted as "primary power" hereinafter) directly from the bus power of the USB. In the case of generating another power supply voltage from the bus power, however, a power supply circuit 17 may be equipped as in the case of the first embodiment. In such a case, the power supply circuit 17 generates a required power supply in the respective circuits of the USB controller 16, communication control unit 15 and insulation element 13 on the basis of the bus power.

The power supply circuit 22a, insulation element 14 and ground-line-use terminal 11d are connected to the ground GND on the secondary side (i.e., the WS 8 side). The insulation element 13, communication control unit 15, USB controller 16, and ground-line-use terminal 18d are connected to the ground GND on the primary side (i.e., the reception device 4 side). As a result, the insulation adaptor 6 is insulated along the part indicated by the dotted line shown in Fig. 4.

The operations of the insulation adaptor 6 when data is transferred from the reception device 4 to WS 8 and when data is transferred from the WS8 to reception apparatus 4 are the same as in the case of the first embodiment.

The present embodiment is configured to supply the reception device with power from the AC power source, and thereby the consumption of a battery can be suppressed. If the reception device is used only when the insulation adaptor is attached, a battery is not required on the reception device.

Further, if a configuration is such that the reception device uses the power of a battery inside of the reception device, a line for the power-supply-use terminal 11e will no longer be required and therefore the number of lines of the exclusive-use cable can be reduced from four to three. As a result, it will no longer be necessary to supply the reception device with the power.

### [Embodiment 2]

Embodiment 2 is described for a method of equipping the inside of an insulation adaptor with an insulated power supply circuit, in place of equipping an AC adaptor with an insulated power supply circuit.

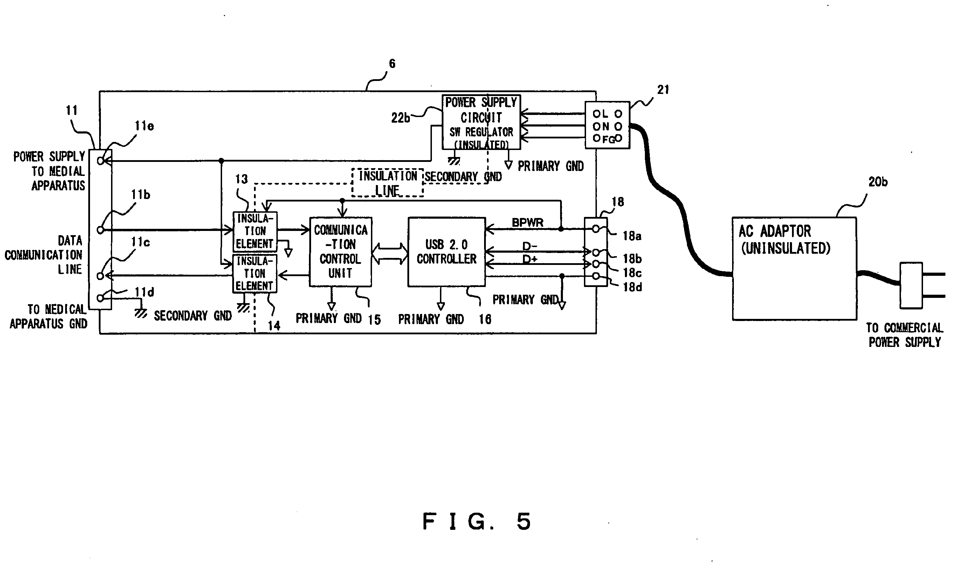

Fig. 5 is a block diagram of an insulation adaptor 6 according to the present embodiment (exemplary embodiment 2).

The differences from embodiment 1 are that the present embodiment uses an uninsulated AC adaptor 20b in place of the AC adaptor 20a and that it uses an insulated power supply circuit 22b (e.g., an insulated switching (SW) regulator or the like), as a power supply circuit on the secondary side, in place of the power supply circuit 22a.

The secondary side of the power supply circuit 22b, the insulation element 14, and the ground-line-use terminal 11d are connected to the ground GND on the secondary side (i.e., the WS 8 side). The primary side of the power supply circuit 22b, the insulation element 13, communication control unit 15, USB controller 16, and ground-line-use terminal 18d are connected to the ground GND on the primary side (i. e., the reception device 4 side). As a result, the insulation adaptor 6 is insulated along the part indicated by the dotted line shown in Fig. 5.

The operations of the insulation adaptor when data is transferred from the reception device 4 to WS 8 and when data is transferred from the WS 8 to reception device 4 are the same as in the case of the first embodiment.

The present embodiment is configured such that the AC power supply supplies the reception device with the power when the insulation adaptor is attached and therefore the consumption of the battery can be suppressed. If the reception device is used only when the insulation adaptor is attached, a battery will no longer be required for the reception device.

Further, configuring such that the reception device uses only the power of a battery inside of the reception device, the line for the power-supply-use terminal 11e is no longer required. Therefore, the number of lines of the exclusive-use cable can be reduced from four to three. As a result, a power supply to the reception device is no longer required.

Further, the insulation is secured within the insulation adaptor and therefore a specially formed connector is no longer required for the AC cable terminal.

### <Third Embodiment>

The present embodiment is described for a cradle-cum-insulation adaptor having a charging function. Note that the same component sign is assigned to the constituent components already described for the first and second embodiments and the description is not provided here.

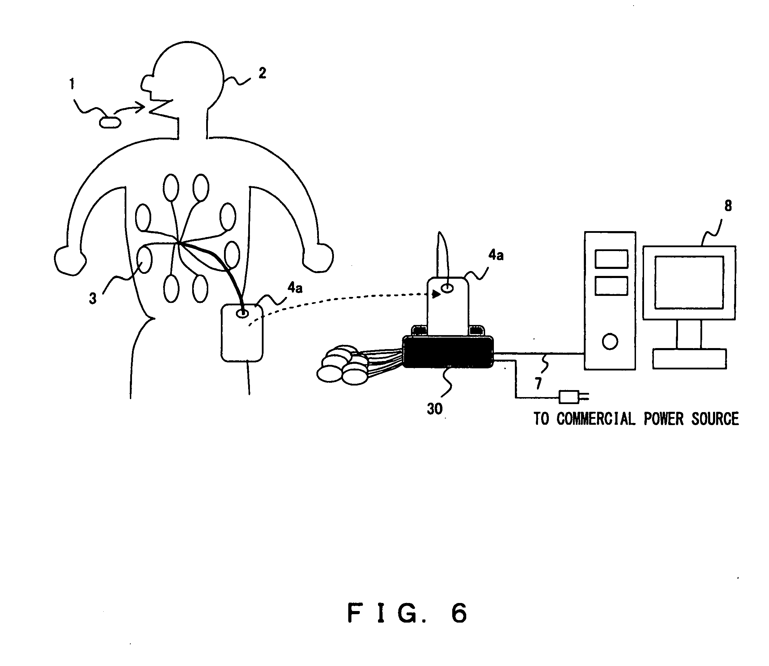

Fig. 6 shows an outline of the connection between a human-body contacting portable medical apparatus and a work station when data is transferred, according to the present embodiment.

As with the other embodiments, the present embodiment is also described for the case of utilizing a reception device used for receiving data picked up by a capsule endoscope as a human-body contacting portable medical apparatus.

A cradle 30 is a cradle having a charging function and adopting the insulation structure described for the first or second embodiment. The cradle 30 is equipped with an AC adaptor and is driven on the basis of the power supplied from a commercial power source. Further, the cradle 30 is connected to the WS 8 via a high-speed communication cable 7. Therefore, the cradle 30 is enabled to transfer data stored in the reception device 4a mounted on the cradle 30 to the WS 8 by way of the high-speed communication cable 7.

The following is a usage method of the cradle 30. After completing an examination by means of a capsule endoscope, the reception device 4a is removed from the patient and mounted onto the cradle 30. This prompts the cradle 30 to transfer the data recorded in the recording medium inside of the reception device 4a to the WS 8. In this event, the cradle 30 also charges the battery inside of the reception device 4a.

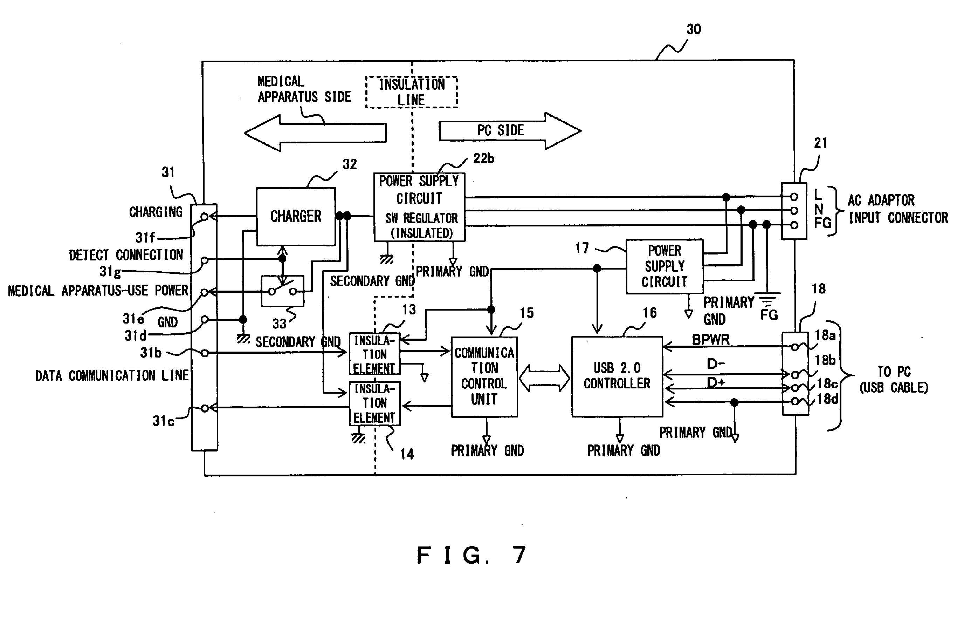

Fig. 7 is a block diagram of a cradle 30 according to the present embodiment. The cradle 30. comprises a cradle connector 31, a charger 32, insulation elements 13 and 14, a communication control unit 15, a USB controller 16, a power supply circuit 17, and a USB cable connector 18.

The cradle connector 31 is a connector used for connecting the cradle 30 to a reception device 4a. The cradle connector 31 is equipped with a charging-use terminal 31f, a connection-detection-use terminal 31g, a power-supply-use terminal 31e, a ground-line-use terminal 31d, a data-communication-line-use input terminal 31b, and a data-communication-line-use output terminal 31c.

The charging-use terminal 31f is a terminal to which the power is supplied for charging the reception device 4a when it is mounted on the cradle 30. The power-supply-use terminal 31e is a terminal for supplying the reception device 4a with the power of the power supply circuit 22b. The ground-line-use terminal 31d is a terminal for connecting the reception device 4a to the ground line. The data-communication-line-use input terminal 31b is a terminal used for a communication line to transfer data to be transmitted from the reception device 4a. The data-communication-line-use output terminal 31c is a terminal used for a communication line to transfer data to be transmitted to the reception device 4a.

Meanwhile, the connection-detection-use terminal 31g is a terminal used for detecting a mounting of the reception device 4a onto the cradle 30. The connection-detection-use terminal 31g has a high voltage when a reception device 4a is not connected. The connection-detection-use terminal 31g has a low voltage when the reception device 4a is connected.

A switch 33 is a switch used for being turned ON/OFF on the basis of the voltage of the connection-detection-use terminal 31g. When the reception device 4a is mounted on the cradle 30, the switch 33 is turned ON on the basis of the voltage of the connection-detection-use terminal 31g. In this event, the power from the power supply circuit 22b is supplied to the reception device 4a by way of the power-supply-use terminal 31e. In contrast, when a reception device 4a is not mounted on the cradle 30, the switch 33 is turned OFF on the basis of the voltage of the connection-detection-use terminal 31g. In this event, the power is not supplied to the power-supply-use terminal 31e. This configuration makes it possible to prevent a shorting of the power-supply-use terminal 31e when the reception device 4a is not connected.

The power supply circuit 17 adjusts the power supplied from the commercial power source by way of the AC adaptor input connector 21 and supplies the insulation element 13, communication control unit 15, and USB controller 16 with the adjusted power. In this event, the USB controller 16 does not use the bus power of the USB.

The power supply circuit 22b is an insulated power supply circuit (e.g., an insulated switching (SW) regulator or the like). The power supply circuit 22b adjusts the power supplied from the commercial power source via the AC adaptor input connector 21 and supplies the insulation element 14 and charger 32 with the adjusted power.

The charger 32 operates on the basis of the voltage of the connection-detection-use terminal 31g and supplies the power for charging the reception device 4a. When the reception device 4a is mounted on the cradle 30, the charger 32 supplies the reception device 4a with power by way of the charging-use terminal 31f on the basis of the voltage of the connection-detection-use terminal 31g. When a reception device 4a is not mounted, the charger 32 does not operate on the basis of the voltage of the connection-detection-use terminal 31g. Accordingly the charging-use terminal 31f is not supplied with the power. This configuration makes it possible to prevent a shorting of the charging-use terminal 31f when a reception device 4a is not connected.

Next is a description of the cradle 30. When the reception device 4a is mounted onto the cradle 30, the voltages at the connection-detection-use terminal 31g are changed from High to Low. This prompts the charger 32 to operate to supply the reception device 4a mounted on the cradle 30 with the power for charging.

Meanwhile, the switch 33 is also switched over from OFF to ON. This prompts the power for driving the reception device 4a, when it is charged, to be supplied from the power supply circuit 22b to the reception device 4a. Thereafter, the operations of the cradle 30 when data is transferred from the reception device 4a to WS 8 and when data is transferred from the WS 8 to reception device 4a are the same as in the case of the first embodiment.

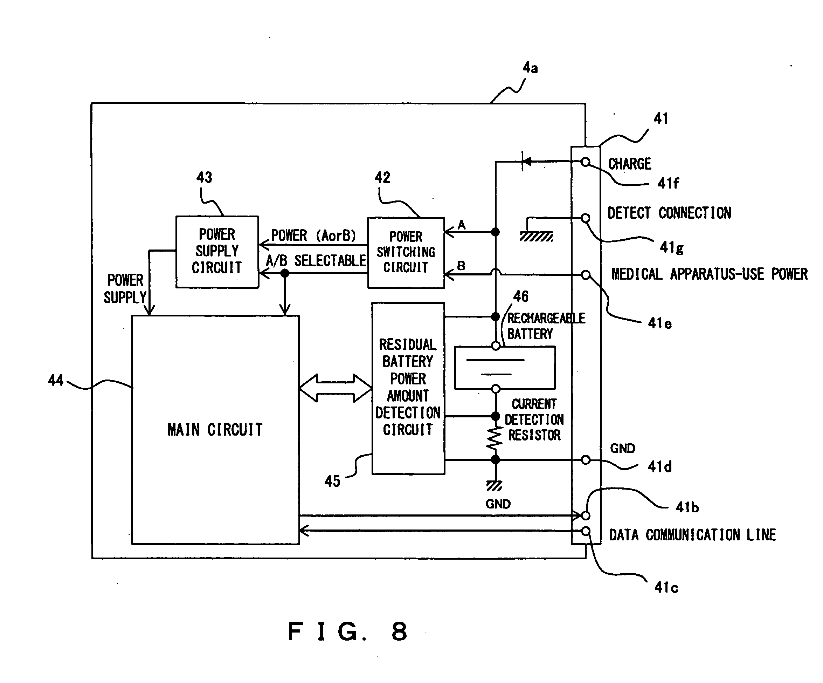

Fig. 8 is a block diagram of the reception device 4a according to the present embodiment. The reception device 4a comprises a cradle connector 41, a power supply switching circuit 42, a power supply circuit 43, a main circuit 44, a residual battery power amount detection circuit 45, and a rechargeable battery 46.

The cradle connector 41 is a connector used for connecting the reception device 4a to the cradle 30. The cradle connector 41 is equipped with a charge-use terminal 41f, a connection-detection-use terminal 41g, a power-supply-use terminal 41e, a ground-line-use terminal 41d, a data-communication-line-use output terminal 41b, and a data-communication-line-use input terminal 41c.

The charge-use terminal 41f is a terminal which is connected to the charging-use terminal 31f of the cradle 30 and to which the power for charging is supplied from the charger 32.

The connection-detection-use terminal 41g is a terminal for changing the voltages of the connection-detection-use terminal 31g from high to low when the connection-detection-use terminal 41g is connected to the power-supply-use terminal 31e of the cradle 30.

The power-supply-use terminal 41e is a terminal which is connected to the power-supply-use terminal 31e in the cradle 30 and which is for supplying the power of driving the reception device 4a from the power supply circuit 22b when charging is performed.

The ground-line-use terminal 41d is a terminal used for connections to the ground via it being connected to the ground-line-use terminal 31d of the cradle 30.

The data communication-line-use output terminal 41b is a communication-line-use terminal which is connected to the data-communication-line-use input terminal 31b of the cradle 30 and which is used for transferring data from the reception device 4a to the cradle 30.

The data-communication-line-use input terminal 41c is a communication-line-use terminal which is connected to the data-communication-line-use output terminal 31c and which is used for transferring data from the cradle 30 to the reception device 4a.

The rechargeable battery 46 is charged with the power supplied from the charger 32 of the cradle 30 via the charge-use terminal 41f.

The power supply switching circuit 42 switches over to the power on the line A side (i.e., the power from the rechargeable battery 46; noted as "power A" hereinafter) when charging is not performed (i.e., when a reception device 4a is not mounted on the cradle 30). Further, the power supply switching circuit 42 switches over to the power on the line B side (i.e., the power from the power supply circuit 22b supplied via the power-supply-use terminal 41e; noted as "power B" hereinafter) when charging is performed (i.e., when the reception device 4a is mounted on the cradle 30). Further, the power supply switching circuit 42 outputs, to the power supply circuit 43 and main circuit 44, a signal indicating which power (i.e., power A or power B) the selected power is.

The power supply circuit 43 adjusts the power (i.e., power A or B) output from the power supply switching circuit 42 on the basis of the signal indicating which of powers A and B the power selected by the power supply switching circuit 42 is and supplies the main circuit 44 with the adjusted power. The configuration is such that the analog-series power supply comprised in the power supply circuit 43 will not be turned ON when the reception device 4a is connected to the cradle 30.

The main circuit 44 switches over the operation content on the basis of the signal indicating which of powers A and B the power selected by the power supply switching circuit 42 is. If the power selected by the power supply switching circuit 42 is power A, the reception device 4a is driven by the rechargeable battery 46, that is, the reception device 4a is removed from the cradle 30. In this case, the main circuit 44 is driven in the normal operation mode (i.e., standing by to receive data from the capsule endoscope 1).

In contrast, if the power selected by the power supply switching circuit 42 is power B, the reception device 4a is mounted on the cradle 30 and is driven by the power supplied from the power supply circuit 22b via the power-supply-use terminal 41e. In this event, the main circuit 44 is driven in a data transfer mode (i.e., the mode for exchanging data between the reception device 4a and WS 8). In the data transfer mode, the main circuit 44 transfers data to WS 8, and receives data from the WS 8 by way of the cradle 30, respectively using the data-communication-line-use output terminal 41b and data-communication-line-use input terminal 41c.

The residual battery power amount detection circuit 45 detects the voltage of the rechargeable battery 46 and outputs the detected voltage, as a residual battery power, to the main circuit 44. The main circuit 44 displays the residual battery power on the display unit of the reception device 4a on the basis of the detected voltage.

Note that the present embodiment is configured to use the insulated power supply circuit 22b; it may alternatively be configured to use an insulated AC adaptor in place of the insulated power supply circuit as in the case of the second embodiment (i.e., exemplary embodiment 1). In such a case, the AC adaptor is equipped with a specially formed connector for the input connector in order to prevent an insertion of another uninsulated adaptor.

The present embodiment enables the reception device to charge while transferring data. The present embodiment is configured to drive the reception device with the AC power when it is mounted on the cradle, thereby making it possible to suppress the power consumed by the rechargeable battery built-in in the reception device.

Note that the present invention can be configured or embodied in various manners possible within the spirit and scope of the present invention, in lieu of being limited to the embodiments put forth in the above description. Further, the present invention may combine the above described first through third embodiments to within a viable extent.

The present invention makes it possible to adjust the speed of communications between a portable medical apparatus and a general purpose PC, and also to secure the electrical insulation between the portable medical apparatus and a general purpose PC.

## Claims

1. An adaptor device (6,30) connected to both a portable medical apparatus and a computer which exchanges data with the portable medical apparatus, comprising:
a first interface (18) which is connected to the computer and which communicates at a first communication speed with the computer;
a second interface (11, 31) which is connected to the portable medical apparatus and which communicates at a second communication speed with the portable medical apparatus; and
a communication control unit (15) for adjusting the speed between a first signal exchanged at the first communication speed by way of the first interface and a second signal exchanged at the second communication speed by way of the second interface;
**characterized in that** the adaptor device further comprises a first insulation unit (13), which is equipped between the second interface (11, 31) and the communication control unit (15) and which electrically insulates between a terminal on the second interface side and a terminal on the communication control unit side, for outputting, to the communication control unit (15), the second signal input from the second interface (11, 31);
a second insulation unit (14), which is equipped between the second interface (11, 31) and the communication control unit (15) and which electrically insulates between a terminal on the second interface side and a terminal on the communication control unit side, for outputting, to the second interface (11, 31), a signal input from the communication control unit (15);
a first power supply unit (17, 18a) for driving the first insulation unit (13) and communication control unit (15); and
a second power supply unit (12, 20a, 22b) which drives the second insulation unit (14) and which is insulated from the first power supply unit (17, 18a).

2. The adaptor device according to claim 1, wherein
the communication control unit (15) converts the communication speed of a second signal input by way of the first insulation unit (13) into the first communication speed that is higher than the second communication speed, outputs the converted communication speed to the first interface, converts the communication speed of a first signal input by way of the first interface (18) into the second communication speed, and outputs the converted communication speed to the second insulation unit (14).

3. The adaptor device according to claim 2, wherein
the first power supply unit is a power supply (17, 18a) based on the bus power of a universal serial bus (USB) if the first interface (18) is an interface based on the USB, and
the second power supply unit is a power supply (12) based on the power supplied from the portable medical apparatus.

4. The adaptor device according to claim 2, wherein
the first power supply unit is a power supply (17, 18a) based on the bus power of a universal serial bus (USB) if the first interface (18) is an interface based on the USB, and
the second power supply unit is an insulated alternate current (AC) adaptor (20a) enabled to supply commercial electric power by being connected to the adaptor device.

5. The adaptor device according to claim 1, wherein
the first power supply unit is a power supply (17, 18a) based on the bus power of a universal serial bus (USB) if the first interface (18) is an interface based on the USB, and
the second power supply unit is an insulated power supply circuit (22b) which adjusts electric power supplied from an alternate current (AC) adaptor to supply the second insulation unit (14) with the adjusted power if the AC adaptor enabled to supply commercial electric power by being connected to the adaptor device is an uninsulated AC adaptor.

6. The adaptor device according to claim 1, wherein
the first insulation unit (13) and the second insulation unit (14) are photo couplers, wherein
the second communication speed is within a range compatible with the photo coupler.

7. The adaptor device according to claim 1, wherein
the first communication speed is a communication speed based on a communication protocol used for a general purpose computer equipped with at least a universal serial bus (USB).

8. The adaptor device according to claim 1, wherein
the adaptor device is a cradle (30) enabled to attach the portable medical apparatus.

9. The adaptor device according to claim 8, further comprising
a charger unit (32) for charging, via the second interface, a rechargeable battery (46) existing inside of the portable medical apparatus, wherein
the charger unit (32) supplies the rechargeable battery with electric power when the portable medical apparatus is attached, and the second power supply unit (12, 20a, 22b) supplies electric power for driving the portable medical apparatus by way of the second interface (11, 31) while the charging is being performed when the portable medical apparatus is attached.

10. A medical apparatus system constituted by a portable medical apparatus (4,4a), a computer (8), and an adaptor device (6,30) according to any one of the preceding claims.

## Patentansprüche

1. Adaptervorrichtung (6, 30), die mit einem tragbaren medizinischen Gerät und mit einem Computer, der Daten mit dem tragbaren medizinischen Gerät austauscht, verbunden ist, umfassend:
eine erste Schnittstelle (18), die mit dem Computer verbunden ist, und die mit einer ersten Kommunikationsgeschwindigkeit mit dem Computer kommuniziert;
eine zweite Schnittstelle (11, 31), die mit dem tragbaren medizinischen Gerät verbunden ist, und die mit einer zweiten Kommunikationsgeschwindigkeit mit dem tragbaren medizinischen Gerät kommuniziert; und
einer Kommunikationssteuereinheit (15) zum Abgleichen der Geschwindigkeit zwischen einem mit der ersten Kommunikationsgeschwindigkeit über die erste Schnittstelle ausgetauschten ersten Signal und einem mit der zweiten Kommunikationsgeschwindigkeit über die zweite Schnittstelle ausgetauschten zweiten Signal;
**dadurch gekennzeichnet, dass** die Adaptervorrichtung ferner umfasst
eine erste Isoliereinheit (13), die zwischen der zweiten Schnittstelle(11, 31) und der Kommunikationssteuereinheit (15) eingerichtet ist, und die zwischen einem Endgerät auf der Seite der zweiten Schnittstelle und einem Endgerät auf der Seite der Kommunikationssteuereinheit elektrisch isoliert, um an die Kommunikationssteuereinheit (15) die zweite Signaleingabe von der zweiten Schnittstelle (11, 31) auszugeben;
eine zweite Isoliereinheit (14), die zwischen der zweiten Schnittstelle(11, 31) und der Kommunikationssteuereinheit (15) eingerichtet ist, und die zwischen einem Endgerät auf der Seite der zweiten Schnittstelle und einem Endgerät auf der Seite der Kommunikationssteuereinheit elektrisch isoliert, um an die zweite Schnittstelle (11, 31) eine Signaleingabe von der Kommunikationssteuereinheit (15) auszugeben;
eine erste Stromversorgungseinheit (17, 18a) zum Antrieb der ersten Isoliereinheit (13) und der Kommunikationssteuereinheit (15); und
eine zweite Stromversorgungseinheit (12, 20a, 22b), welche die zweite Isoliereinheit (14) antreibt und von der ersten Stromversorgungseinheit (17, 18a) isoliert ist.

2. Adaptervorrichtung nach Anspruch 1, wobei
die Kommunikationssteuereinheit (15) die Kommunikationsgeschwindigkeit einer zweiten Signaleingabe über die erste Isoliereinheit (13) in die erste Kommunikationsgeschwindigkeit, die höher ist als die zweite Kommunikationsgeschwindigkeit, umwandelt, die umgewandelte Kommunikationsgeschwindigkeit an die erste Schnittstelle ausgibt, die Kommunikationsgeschwindigkeit einer ersten Signaleingabe über die erste Schnittstelle (18) in die zweite Kommunikationsgeschwindigkeit umwandelt, und die umgewandelte Kommunikationsgeschwindigkeit an die zweite Isoliereinheit (14) ausgibt.

3. Adaptervorrichtung nach Anspruch 2, wobei
die erste Stromversorgungseinheit eine Stromversorgung (17, 18a) ist, die auf der Bus-Energie eines Universal Serial Bus (USB) basiert, falls die erste Schnittstelle (18) eine auf dem USB basierende Schnittstelle ist, und
die zweite Stromversorgungseinheit eine Stromversorgung (12) ist, die auf der von dem tragbaren medizinischen Gerät gelieferten Energie basiert.

4. Adaptervorrichtung nach Anspruch 2, wobei
die erste Stromversorgungseinheit eine Stromversorgung (17, 18a) ist, die auf der Bus-Energie eines Universal Serial Bus (USB) basiert, falls die erste Schnittstelle (18) eine auf dem USB basierende Schnittstelle ist, und
die zweite Stromversorgungseinheit ein isolierter Wechselstrom(AC)-Adapter (20a) ist, der elektrischen Netzstrom liefern kann, indem er mit der Adaptervorrichtung verbunden wird.

5. Adaptervorrichtung nach Anspruch 1, wobei
die erste Stromversorgungseinheit eine Stromversorgung (17, 18a) ist, die auf der Bus-Energie eines Universal Serial Bus (USB) basiert, falls die erste Schnittstelle (18) eine auf dem USB basierende Schnittstelle ist, und
die zweite Stromversorgungseinheit eine isolierte Stromversorgungsschaltung (22b) ist, welche von einem Wechselstrom(AC)-Adapter gelieferte elektrische Energie so anpasst, dass die zweite Isoliereinheit (14) mit der angepassten Energie versorgt wird, falls der AC-Adapter, der elektrischen Netzstrom liefern kann, indem er mit der Adaptervorrichtung verbunden wird, ein nicht-isolierter AC-Adapter ist.

6. Adaptervorrichtung nach Anspruch 1, wobei
die erste Isoliereinheit (13) und die zweite Isoliereinheit (14) Photokoppler sind, wobei
die zweite Kommunikationsgeschwindigkeit in einem mit dem Photokoppler kompatiblen Bereich liegt.

7. Adaptervorrichtung nach Anspruch 1, wobei
die erste Kommunikationsgeschwindigkeit eine Kommunikationsgeschwindigkeit ist, die auf einem Kommunikationsprotokoll basiert, das für einen mit mindestens einem Universal Serial Bus (USB) ausgestatteten Allzweck-Computer verwendet wird.

8. Adaptervorrichtung nach Anspruch 1, wobei
die Adaptervorrichtung eine Ladestation (30) ist, an die das tragbare medizinische Gerät angeschlossen werden kann.

9. Adaptervorrichtung nach Anspruch 8, ferner mit
einer Ladeeinheit (32) zum Laden, über die zweite Schnittstelle, einer in dem tragbaren medizinischen Gerät vorhandenen, wiederaufladbaren Batterie (46), wobei
die Ladeeinheit (32) die wiederaufladbare Batterie mit elektrischer Energie versorgt, wenn das tragbare medizinische Gerät angeschlossen ist, und die zweite Stromversorgungseinheit (12, 20a, 22b) elektrische Energie zum Antrieb des tragbaren medizinischen Geräts über die zweite Schnittstelle (11, 31) liefert, während der Ladevorgang erfolgt, wenn das tragbare medizinische Gerät angeschlossen ist.

10. Medizinisches Gerätesystem, bestehend aus einem tragbaren medizinischen Gerät (4, 4a), einem Computer (8) und einer Adaptervorrichtung (6, 30) nach einem der vorangehenden Ansprüche.

## Revendications

1. Dispositif adaptateur (6, 30) connecté à la fois à un appareil médical portable et à un ordinateur qui échange des données avec l'appareil médical portable, comprenant :
une première interface (18) qui est connectée à l'ordinateur et qui communique à une première vitesse de communication avec l'ordinateur ;
une seconde interface (11, 31) qui est connectée à l'appareil médical portable et qui communique à une seconde vitesse de communication avec l'appareil médical portable ; et
une unité de commande de communication (15) destinée à régler la vitesse entre un premier signal échangé à la première vitesse de communication par le biais de la première interface et un second signal échangé à la seconde vitesse de communication par le biais de la seconde interface ;
**caractérisé en ce que** le dispositif adaptateur comprend en outre :
une première unité d'isolation (13) qui est montée entre la seconde interface (11, 31) et l'unité de commande de communication (15) et qui procure une isolation électrique entre une borne du côté de la seconde interface et une borne du côté de l'unité de commande de communication, pour délivrer en sortie, à l'unité de commande de communication (15), le second signal appliqué à partir de la seconde interface (11, 31) ;
une seconde unité d'isolation (14) qui est montée entre la seconde interface (11, 31) et l'unité de commande de communication (15) et qui procure une isolation électrique entre une borne du côté de la seconde interface et une borne du côté de l'unité de commande de communication, pour délivrer en sortie, à la seconde interface (11, 31), un signal appliqué à partir de l'unité de commande de communication (15) ;
une première unité d'alimentation (17, 18a) destinée à commander la première unité d'isolation (13) et l'unité de commande de communication (15) ; et
une seconde unité d'alimentation (12, 20a, 22b) qui commande la seconde unité d'isolation (14) et qui est isolée de la première unité d'alimentation (17, 18a).

2. Dispositif adaptateur selon la revendication 1, dans lequel
l'unité de commande de communication (15) convertit la vitesse de communication d'un second signal appliqué par le biais de la première unité d'isolation (13) dans la première vitesse de communication qui est supérieure à la seconde vitesse de communication, délivre en sortie la vitesse de communication convertie à la première interface, convertit la vitesse de communication d'un premier signal appliqué par le biais de la première interface (18) dans la seconde vitesse de communication, et délivre en sortie la vitesse de communication convertie à la seconde unité d'isolation (14).

3. Dispositif adaptateur selon la revendication 2, dans lequel
la première unité d'alimentation est une unité d'alimentation (17, 18a) fondée sur la puissance de bus d'un bus série universel (USB) si la première interface (18) est une interface fondée sur l'USB, et
la seconde unité d'alimentation est une alimentation (12) fondée sur l'énergie fournie à partir de l'appareil médical portable.

4. Dispositif adaptateur selon la revendication 2, dans lequel
la première unité d'alimentation est une alimentation (17, 18a) fondée sur la puissance de bus d'un bus série universel (USB) si la première interface (18) est une interface fondée sur l'USB, et
la seconde unité d'alimentation est un adaptateur de courant alternatif (CA) isolé (20a) apte à fournir de l'énergie électrique commerciale en étant connecté au dispositif adaptateur.

5. Dispositif adaptateur selon la revendication 1, dans lequel
la première unité d'alimentation est une d'alimentation (17, 18a) fondée sur la puissance de bus d'un bus série universel (USB) si la première interface (18) est une interface fondée sur l'USB, et
la seconde unité d'alimentation est un circuit d'alimentation isolé (22b) qui règle l'énergie électrique fournie à partir d'un adaptateur de courant alternatif (CA) pour alimenter la seconde unité d'isolation (14) avec la puissance réglée si l'adaptateur CA apte à fournir de l'énergie électrique commerciale en étant connecté au dispositif adaptateur est un adaptateur CA non isolé.

6. Dispositif adaptateur selon la revendication 1, dans lequel
la première unité d'isolation (13) et la seconde unité d'isolation (14) sont des photocoupleurs, dans lequel
la seconde vitesse de communication se situe dans une plage compatible avec le photocoupleur.

7. Dispositif adaptateur selon la revendication 1, dans lequel
la première vitesse de communication est une vitesse de communication fondée sur un protocole de communication utilisé pour un ordinateur universel équipé au moins d'un bus série universel (USB).

8. Dispositif adaptateur selon la revendication 1, dans lequel
le dispositif adaptateur est une station d'accueil (30) apte à fixer l'appareil médical portable.

9. Dispositif adaptateur selon la revendication 8, comprenant en outre
une unité formant chargeur (32) destinée à charger, via la seconde interface, une batterie rechargeable (46) se trouvant à l'intérieur de l'appareil médical portable, dans lequel
l'unité formant chargeur (32) alimente la batterie rechargeable en énergie électrique lorsque l'appareil médical portable est fixé, et la seconde unité d'alimentation (12, 20a, 22b) fournit de l'énergie électrique pour commander l'appareil médical portable par le biais de la seconde interface (11, 31) pendant que la charge s'effectue lorsque l'appareil médical portable est fixé.

10. Système d'appareil médical constitué d'un appareil médical portable (4, 4a), d'un ordinateur (8) et d'un dispositif adaptateur (6, 30) selon l'une quelconque des revendications précédentes.
